# EUROPÄISCHE PATENTANMELDUNG

(11) **EP 3 085 412 A1**
(43) Veröffentlichungstag der Anmeldung: **26.10.2016**
(21) Anmeldenummer: 16161307.0
(22) Anmeldetag: 21.03.2016
(51) Int. Cl.: A61N 1/05

(54) **IMPLANTIERBARES GEBOGENES FORMGEBUNGSTEIL ZUR ÄUSSEREN FORMUNG EINER IMPLANTIERBAREN ELEKTRODENLEITUNG ODER EINES KATHETERS**

(30) Priorität: 20.04.2015 US 201562149675 P
(71) Anmelder: BIOTRONIK SE & Co. KG, 12359 Berlin (DE)
(72) Erfinder: Jadwizak, Detmar, 15537 Erkner (DE); Fründt, Carsten, 13057 Berlin (DE); Kaiser, Dajana, 12247 Berlin (DE); Hillebrand, Gordon, 12157 Berlin (DE)
(74) Vertreter: Galander, Marcus

(57) **Zusammenfassung**

Implantierbares gebogenes Formgebungsteil zur äußeren Formung einer implantierbaren Elektrodenleitung oder eines Katheters, wobei das Formgebungsteil ein durchgehendes erstes Lumen zum Durchgang eines Abschnitts der Elektrodenleitung oder des Katheters hat, wobei das Formgebungsteil als Spritzgussteil ausgebildet ist oder mindestens einen Spritzguss-Abschnitt aufweist und innerhalb der Wandung oder an der Innenwand ein langgestrecktes, steifes aber biegsames Biegungs-Einprägeelement fixiert ist.

## Beschreibung

Die Erfindung betrifft ein implantierbares gebogenes Formgebungsteil zur äußeren Formung einer implantierbaren Elektrodenleitung oder eines Katheters, wobei das Formgebungsteil ein durchgehendes erstes Lumen zum Durchgang eines Abschnitts der Elektrodenleitung oder des Katheters hat. Sie betrifft des Weiteren eine Elektrodenleitungs- oder Katheteranordnung, die mit mindestens einem solchen Formgebungsteil gebildet ist.

Implantierte medizinische Elektrodenleitungen (kurz auch als "Elektroden" bezeichnet) müssen im Körper des Patienten an einer geeigneten Stelle positioniert und in dieser Position auch hinreichend dauerhaft fixiert werden, um die gewünschten therapeutischen Wirkungen zuverlässig und dauerhaft zu erzielen. Es hat daher seit der Einführung der ersten implantierbaren Herzschrittmacher eine Vielzahl unterschiedlicher Entwicklungen zur Lösung dieser Aufgabe gegeben.

Zur positionsgetreuen Fixierung von Elektrodenleitungen in Gefäßabschnitten bzw. relativ engen Hohlorganen wurden bereits vor langem Elektrodenleitungen mit eingeprägten Krümmungen im distalen Endbereich vorgeschlagen, die sich nach der Implantation und dem Entfernen des Führungsdrahtes (Guide Wire) oder des Mandrins (Stylet) aufgrund Ihres gekrümmten Verlaufes gewissermaßen zwischen gegenüberliegenden Wandungen des Gefäßes oder Hohlorgans verspannen. Hierdurch kann eine recht verlässliche und auch langzeitstabile Positionsfixierung erreicht werden. Ein Beispiel einer solchen Konstruktion ist beschrieben in US 5,925,073.

In diesem Zusammenhang sind auch Elektrodenleitungen mit speziellen inneren Strukturen entwickelt worden, mit denen eine über den Längsverlauf variable Steifigkeit bzw. Biegsamkeit realisiert werden soll; vgl. hierzu etwa US 6,556,873 B1 oder EP 2 024 014 B1.

Speziell sogenannte CRT-Elektroden werden in Koronarsinusgefäßen im Bereich des linken Ventrikels implantiert. Dazu besitzen diese Elektroden eine "passive" Fixierung der oben erwähnten Art, indem der distale Bereich der Elektrode mit einer oder mehreren Krümmungen versehen ist. Bei der Implantation wird diese Krümmung durch einen innen liegenden Mandrin gerade gestellt. Wird dieser Mandrin zurückgezogen, übt die sich wieder krümmende Elektrode Kraft auf die Gefäßinnenwand aus und verankert somit die Elektrode an ihrem gewünschten Ort.

Aus technologischer Sicht sind im Wesentlichen folgende Realisierungsmöglichkeiten einer solchen Lösung bekannt und in Gebrauch:
1. Erzeugen einer Elektrodenkrümmung durch Glühen einer im Leitungskörper liegenden MP35N Wendel.
2. Erzeugen einer Elektrodenkrümmung durch mechanische Verformung (Kaltverformung) einer im Leitungskörper liegenden Wendel. Das wird vorzugsweise bei Coradialwendeln angewandt, da hier jeder Einzeldraht eine Isolationsschicht besitzt, deren Schmelzpunkt unter der Glühtemperatur der Wendel liegt, sodass ein Glühprozess nicht angewendet werden kann.
3. Erzeugen einer Elektrodenkrümmung durch thermisches Verformen von Kunststoffisolationsschläuchen.
4. Erzeugen einer Elektrodenkrümmung durch ein in gekrümmter Form spritzgegossenes Silikonteil.
5. Erzeugen einer Elektrodenkrümmung durch ein Silikonspritzgussteil mit Spannband.

Alle diese Lösungen haben gewisse Nachteile, von denen hier die folgenden genannt werden sollen:
Zu 1.: Bei Verwendung einer ETFE beschichteten Coradialwendel kann kein Glühverfahren angewandt werden. Um mehrere Pole in einem kleinen Durchmesser zu realisieren, ist die Verwendung einer solchen Coradialwendel aber erforderlich.
Zu 2.: Allein durch eine mechanische Verformung der Wendel werden nicht der gewünschte Krümmungswinkel und die gewünschte Krümmungskraft erzeugt.
Zu 3.: Bei Verwendung von Silikon kann keine nachträgliche, thermische Verformung realisiert werden. Silikon ist ein endvernetztes Material, welches sich nach dem Vulkanisierungsprozess nicht mehr umformen lässt. Im distalen Bereich einer CRT-Elektrode ist die Verwendung von Silikon von großem Vorteil, da Flexibilität und Dauerfestigkeit einem Kunststoffmaterial für diese Anwendung überlegen sind.
Zu 4.: Ein Silikonspritzgussteil, welches krumm gespritzt ist, erzeugt nicht den gewünschten Krümmungswinkel und die gewünschte Krümmungskraft (Rückstellkraft).
Zu 5.: Aufgrund des kleinen Durchmessers einer Elektrodenleitung steht für ein Spannband eine nur sehr kleine Wandstärke zur Verfügung. Des Weiteren ist ein Silikonspritzgussteil mit Spannband nur in einem komplizierten und aufwendigen Herstellungsverfahren mit verhältnismäßig hohem Fehlerpotential herstellbar.

Es ist daher Aufgabe der Erfindung, eine verbesserte Lösung zur Realisierung einer in einem Gefäß oder relativ engen Hohlorgan passiv fixierbaren Elektrodenleitung (oder auch eines Katheters) bereitzustellen, die insbesondere zuverlässig und dauerhaft funktioniert, bei der Implantation leicht zu handhaben ist und auch unter Kostenaspekten vertretbar ist.

Es ist, gemäß grundlegenden Überlegungen der Erfinder, ein Silikonteil zu entwickeln, welches bei einem kleinen Durchmesser, geringer Wandstärke, geringer Länge und einfachem Herstellungsverfahren die Funktion der Krümmung einer Coradialwendel und damit die Fixierung der Elektrode Gefäß oder Hohlorgan (speziell einem im Koronargefäß) übernimmt. Dieses Silikonteil muss flexibel, geradestellbar und dauerfest sein sowie eine möglichst hohe Rückstellkraft aufweisen.

Diese Aufgabe wird gelöst durch ein implantierbares gebogenes Formgebungsteil mit den Merkmalen des Anspruchs 1. Zweckmäßige Fortbildungen des Erfindungsgedankens sind Gegenstand der abhängigen Ansprüche. Es werden des Weiteren eine Elektrodenleitungsanordnung mit den Merkmalen des Anspruchs 11 und eine Katheteranordnung mit den Merkmalen des Anspruchs 14 bereitgestellt.

Zur Erfindung gehört in Anbetracht der einerseits äußerst vorteilhaften, unter anderen Aspekten aber unzureichenden Eigenschaften von weichem Silikon der Gedanke der Integration eines die Formgebungskraft des Elementes auf die darin aufgenommene Elektrodenoder Katheterleitung verstärkenden Elementes in das Silikonteil. Dieses wird nachfolgend synonym als "Biegungs-Einprägeelement" oder "kraftverstärkendes Element" bezeichnet. Es ist innerhalb der Wandung oder an der Innenwand des Formgebungsteils fixiert, welches als Spritzgussteil ausgebildet ist oder mindestens einen Spritzguss-Abschnitt aufweist.

In einer Ausführung der Erfindung ist das Formgebungsteil ausgebildet als im Gebrauchszustand U-förmiges oder V-förmiges Teil mit verrundeter Spitze. Als Gebrauchszustand ist der Zustand mit im Formgebungsteil aufgenommener Elektroden- oder Katheterleitung oder versteifendem Führungsdraht (Guide Wire) oder Mandrin (Stylet) zu verstehen. Je nach Länge des Formgebungsteils kann dessen eigene Grundform (ohne Elektroden- oder Katheterleitung) durchaus ein Kreissegment von mehr als 180° umfassen, ja sogar ansatzweise eine Wendel- bzw. Helixform haben. Bevorzugt sind die Enden des Formgebungsteils im Wesentlichen gestreckt ausgebildet.

In einer weiteren Ausführung ist das Spritzgussteil als Silikonteil mit geringer Shore-Härte, insbesondere von 50 Shore oder weniger, ausgebildet. Es ist ein wesentlicher Vorteil der Erfindung, dass durch den Einsatz des "kraftverstärkenden Elementes" ein solch weiches und unter vielen Gesichtspunkten vorteilhaftes Silikonmaterial verwendet werden kann. Im Allgemeinen könnten aber auch Silikonmassen mit höheren Shore-Härten verwendet werden (z.B. bis 80 Shore).

In einer weiteren Ausführung ist an beiden Enden das erste Lumen in seinem Durchmesser derart aufgeweitet, dass Platz für eine innenliegende Klebstoffschicht geschaffen ist. Für den praktischen Einsatz der in Rede stehenden Elektrodenleitungs- oder Katheteranordnungen ist, schon unter Zulassungs-Aspekten, eine feste, stoffschlüssige Verbindung des Formgebungsteils mit einem vorbestimmten Abschnitt der Elektroden- oder Katheterleitung (insbesondere angrenzend an einen Elektrodenpol) zu schaffen. Um dies ohne äußerliche (und somit im Einsatz hinderliche) Durchmesservergrößerung zu erreichen, ist an der Innenwandung des Formgebungsteils Platz für eine (insbesondere ringförmige) Klebstoff schicht zu schaffen.

In weiteren Ausführungen der Erfindung ist das Formgebungsteil ausgebildet als extrudierter Silikonschlauch mit umspritzten Enden, die den aufgeweiteten Querschnitt des ersten Lumens bestimmen. Dieser Silikonschlauch hat mindestens ein zweites Lumen, in dem das Biegungs-Einprägeelement angeordnet ist. Das zweite Lumen ist hierbei insbesondere über die gesamte Länge des Formgebungsteils durchgehend; alternativ ist es aber auch möglich, ein nur von einem Ende her offenes zweites Lumen vorzusehen. In jedem Fall ist die Erstreckung des zweiten Lumens derart zu wählen, dass die Biegungs-Einprägende bzw. kraftverstärkende Funktion hinreichend erzielt werden kann. Im Normalfall sollte das zweite Lumen sich also jedenfalls über den weitaus größeren Teil der Länge des Formgebungsteils erstrecken.

In weiteren Ausführungen der Erfindung ist das Biegungs-Einprägeelement ein federnd steifer gebogener Kunststoffstab. Die Steifigkeit und Federwirkung bzw. Elastizitätseigenschaften des Stabes sind derart auf die mechanischen Eigenschaften eines im konkreten Anwendungsfall im Implantationsvorgang eingesetzten Führungsdrahtes (Guide Wire) oder Mandrins (Stylet) abzustimmen, dass das Formgebungsteil sich dem Verlauf des Führungsdrahtes oder Mandrins weitestgehend anpasst. Zugleich sind die Eigenschaften der Elektrodenleitung (ohne Führungsdraht oder Mandrin) insoweit zu berücksichtigen, dass das Biegungs-Einprägeelement "stärker" als die Elektroden- bzw. Katheterleitung ist, also dieser die gewünschte Krümmung in elastischer Weise verleihen kann.

In einer speziellen Ausgestaltung der erwähnten Ausführung kann vorgesehen sein, dass das Biegungs-Einprägeelement ein Kunststoffstab oder -streifen aus einem Memory-Material ist. Bei dieser Ausgestaltung kommt es nicht im gleichen Maße wie oben erwähnt auf eine Abstimmung der mechanischen Eigenschaften zwischen Elektrodenleitungskörper, Führungsdraht bzw. Mandrin und Formgebungsteil an, weil sich die kraftverstärkende bzw. biegungs-einprägende Wirkung des Stabes erst nach dessen Übergang in die Memory-Form ergibt.

In einer weiteren Ausführung ist das Biegungs-Einprägeelement ein vorgespannter, durch Tempern mit einer Krümmung versehener Kunststoffstab oder -streifen. Für einen solchen Kunststoffstab oder -streifen gelten weitgehend die weiter oben erläuterten Randbedingungen hinsichtlich seiner Steifigkeit und Elastizität.

In weiteren Ausführungen umfasst das Formgebungsteil mehrere Biegungs-Einprägeelemente, die insbesondere jeweils in einem vorgeformten zweiten Lumen angeordnet sind. Im Hinblick auf den erhöhten Entwicklungs- und Herstellungsaufwand erscheinen derartige Ausführungen aus heutiger Sicht zwar nicht als bevorzugt, sie könnten aber zur Erzielung spezieller Krümmungs-Verläufe und/oder eines vorbestimmten Verhaltens während des Implantationsvorganges Vorteile bieten.

In einer Ausführung der vorgeschlagenen Elektrodenleitungsanordnung ist vorgesehen, dass das Formgebungsteil einen Abschnitt zwischen zwei Elektrodenpolen vollständig umgibt, derart, dass ein erstes Ende des Formgebungsteils direkt an einen ersten Elektrodenpol anstößt und ein zweites Ende des Formgebungsteils direkt an einen zweiten Elektrodenpol anstößt. Hiermit wird den weiter oben angesprochenen Anforderungen an die dauerhafte Zuverlässigkeit, speziell zum mechanischen Schutz, zur Isolation und Zugkraftübertragung, die sich auch in Zulassungserfordernissen ausdrücken, Rechnung getragen.

In weiteren Ausführungen der Elektrodenleitungsanordnung sind zwei oder mehr U-förmige oder V-förmige Formgebungsteile in Längsrichtung hintereinander auf der Elektrodenleitung mit entgegengerichteter Krümmung derart angeordnet, dass ein distaler Endabschnitt der Elektrodenleitung insgesamt ein im Wesentlichen S-förmiger, Z-förmiger, J-förmiger, wellenförmiger oder zickzackförmiger Verlauf aufgeprägt hat.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1A bis 1C:: perspektivische Darstellungen bzw. eine perspektivische Schnittansicht zu einem ersten Ausführungsbeispiel der Erfindung,
- Fig. 2A bis 2C: perspektivische Darstellungen bzw. eine perspektivische Schnittansicht zu einem zweiten Ausführungsbeispiel der Erfindung,
- Fig. 3A bis 3E: perspektivische Darstellungen bzw. eine perspektivische Schnittansicht zu einem dritten Ausführungsbeispiel der Erfindung,
- Fig. 4A bis 4D: perspektivische Darstellungen bzw. Längsschnittdarstellungen zu einem vierten Ausführungsbeispiel der Erfindung sowie
- Fig. 5A und 5B: perspektivische Darstellungen zweier Ausführungen einer erfindungsgemäßen Elektrodenleitungsanordnung.

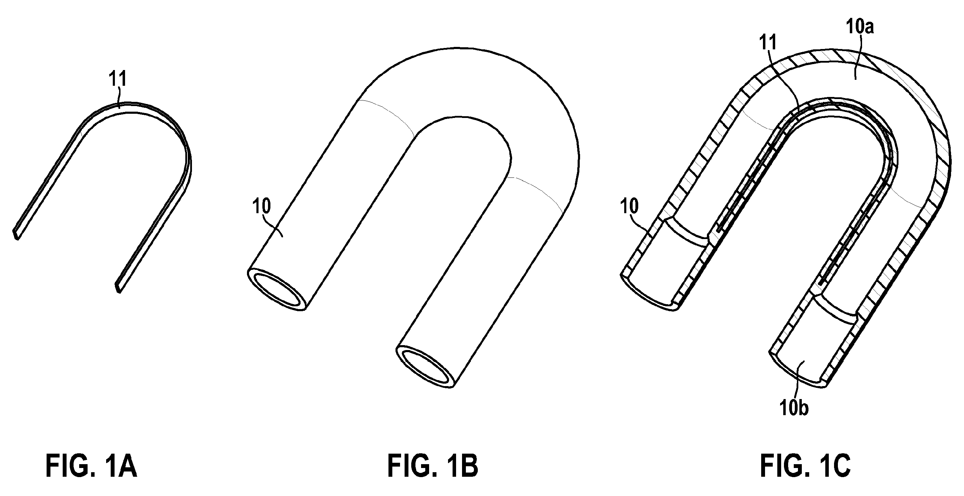

Die Figuren 1B und 1C zeigen ein Formgebungsteil 10 gemäß einem ersten Ausführungsbeispiel der Erfindung, und Fig. 1A zeigt ein U-förmiges steif-elastisches Kunststoffteil 11, z.B. mit kreisförmigem oder rechteckigem Querschnitt, welches als Biegungs-Einprägeelement in das Formgebungsteil 10 integriert ist.
Das Formgebungsteil 10 wird dadurch hergestellt, dass das U-förmige Kunststoffteil 11 in einer geeigneten Spritzgießform mit einem weichen Silikon umspritzt wird. Beim Umspritzen entstehen zugleich die mit ihrem Innendurchmesser aufgeweiteten Endabschnitte 10b sowie das zentrale Lumen 10a. Innere und äußere Mantelschichten des Formgebungsteils 10 werden somit in einem Spritzgießvorgang hergestellt.

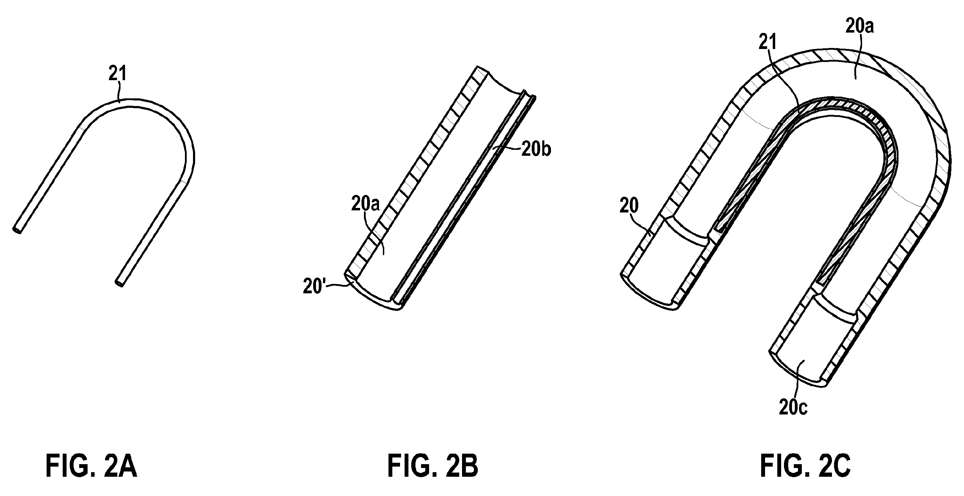

Fig. 2C zeigt als weiteres Ausführungsbeispiel ein zweites Formgebungsteil 20, und Fig. 2B zeigt als zugehöriges Vorprodukt bzw. Halbfabrikat einen extrudierten Silikonschlauch 20' mit einem zentralen ersten Lumen 20a und einem in der Wandung angeordneten zweiten Lumen 20b mit wesentlich geringerem Durchmesser. Fig. 2A zeigt einen steifelastischen Kunststoffstab 21 mit kreisförmigem oder rechteckigem Querschnitt, der in das zweite Lumen 20b des Silikonschlauchs 20' mit kreis- oder halbkreisförmigem Querschnitt eingeführt wird und diesem als Biegungs-Einprägeelement insgesamt eine U-Form verleiht. Wie in Fig. 2C zu erkennen, wird das Formgebungsteil 20 durch Umspritzen des extrudierten Kunststoffschlauches endseitig verlängert, wobei wiederum aufgeweitete Endabschnitte 20c des zentralen Lumens 20a gebildet werden.

In seiner äußeren Gestalt ist das Formgebungsteil 20 gemäß Fig. 2C grundsätzlich nicht vom Formgebungsteil 10 gemäß Fig. 1B und 1C zu unterscheiden; der wesentliche Unterschied besteht darin, dass beim zweiten Formgebungsteil 20 das Biegungs-Einprägeelement in ein längs durchgehendes zweites Lumen des ursprünglichen Silikonschlauches eingeführt wird, während das erste Formgebungsteil 10 in die Spritzgießform eingelegt und erst beim anschließenden Umspritzen in die Wandung des Teils eingebettet wird.

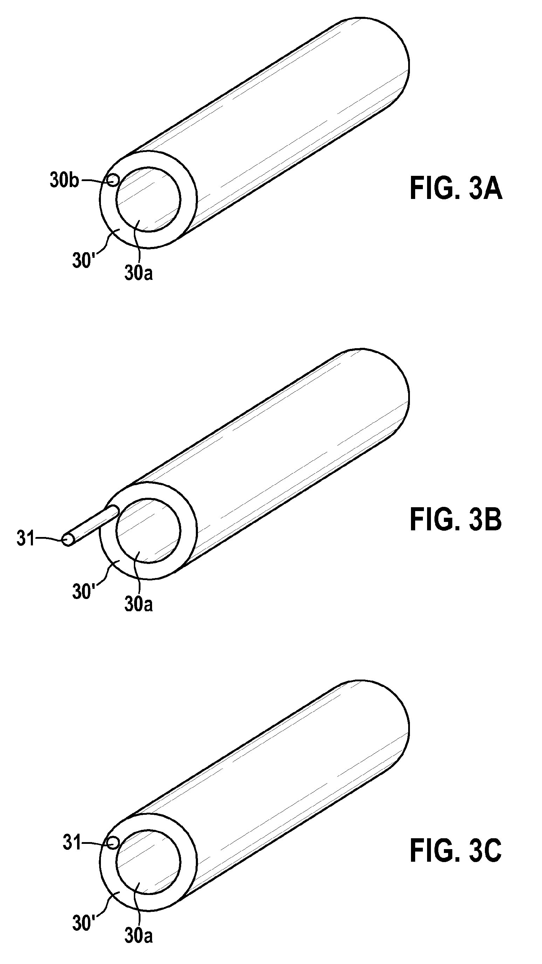

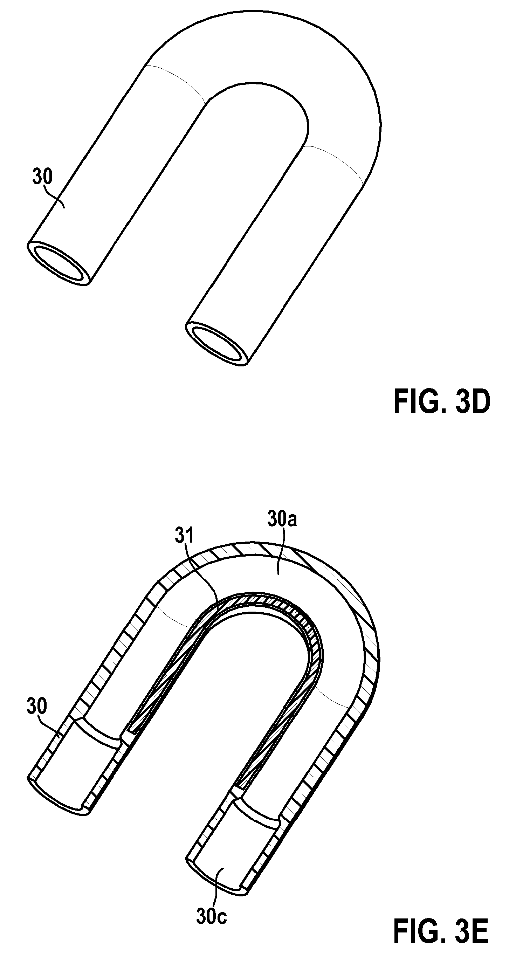

Grundsätzlich ähnlich aufgebaut ist auch das in Fig. 3D und 3E gezeigte Formgebungsteil 30 gemäß einem dritten Ausführungsbeispiel, dessen geradlinig gestrecktes Vorprodukt 30' in Fig. 3A bis 3C dargestellt ist. Auch hier kommt als Vorprodukt ein Silikonschlauch mit einem zentralen Lumen 30A großen Durchmessers und einem in der Wandung angeordneten zweiten Lumen 30b mit wesentlich geringerem Durchmesser zum Einsatz. In das zweite Lumen 30b wird, wie in Fig. 3B und 3C gezeigt, ein in seiner Ausgangsform ebenfalls geradlinig gestreckter Kunststoffstreifen 31 eingeführt und nach Spannen verankert wird. Hier ist es ebenfalls möglich ein halbkreisförmiges Lumen mit einem Kunststoffstreifen (rechteckiger Querschnitt) zu verwenden.

Bei einem nachfolgenden Tempern des Silikonschlauches zieht sich der an beiden Enden des Vorprodukts 30' fixierte Kunststoffstreifen 31 derart zusammen, dass er den gesamten Schlauch in eine U-Form mitzieht. In diesem Zustand wird der Silikonschlauch dann wieder durch Spritzgießen mit Enden mit Aufweitungs-Abschnitten 30c des zentralen Lumens 30a versehen. Wie bei den vorgenannten Ausführungen erwähnt, kann dabei auch eine zusätzliche Silikon-Mantelschicht über den gesamten Schlauch gelegt werden. Dies ist aber lediglich optional vorgesehen, die Enden können auch stumpf an den extrudierten Schlauch anstoßen.

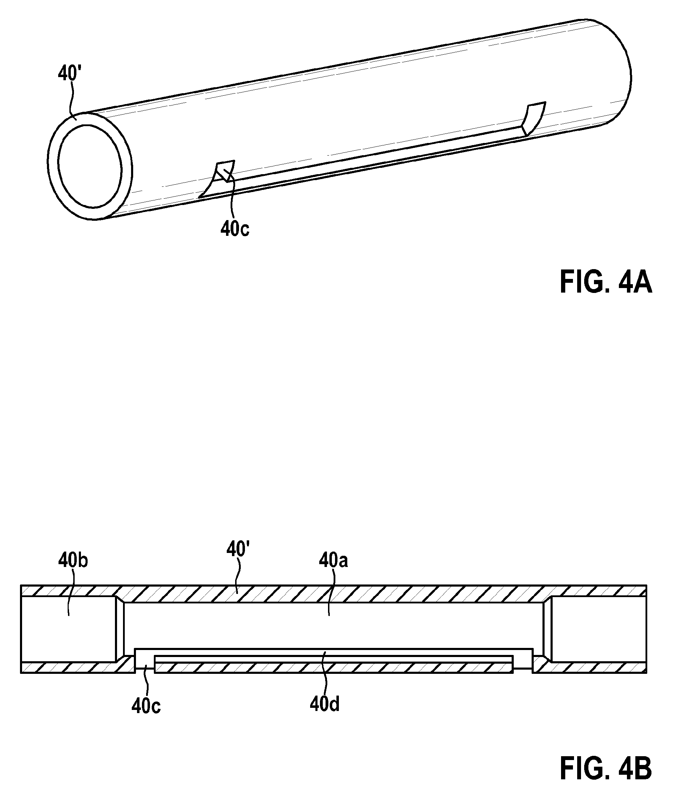

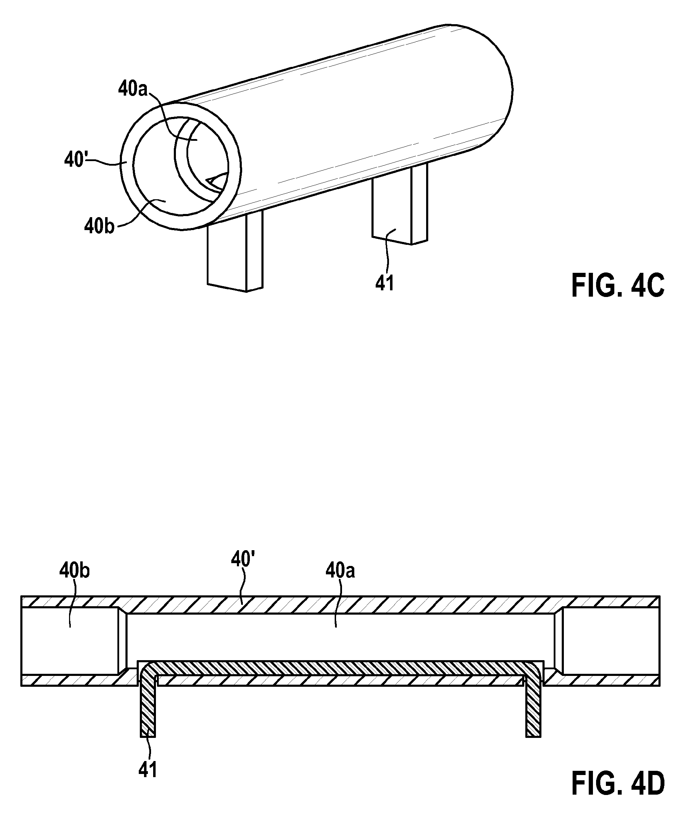

Fig. 4A bis 4D zeigen jeweils Vorstufen 40' eines weiteren Formgebungsteils, das nicht im Endzustand dargestellt ist, dessen endgültige Form aber im Wesentlichen der Form des ersten bis dritten Formgebungsteils entspricht. Hier handelt es sich allerdings nicht, wie bei einigen vorgenannten Ausführungsbeispielen, um einen extrudierten Silikonschlauch, sondern um ein Silikon-Spritzgussteil mit von vorneherein angeformten Endabschnitten mit aufgeweitetem zentralem Lumen 40b.

Über entsprechende Zugänge 40c an einer Seite der Wandung wird in eine hierfür vorgesehene, längs verlaufende Freimachung (Nut) 40d ein Kunststoffstreifen 41 als Biegungs-Einprägeelement bzw. kraftverstärkendes Element eingefügt. Dieses Biegungs-Einprägeelement 41 ist weitgehend plastisch verformbar und kann aus dem in Fig. 4D gezeigten gestreckten Zustand in eine U-Form des mittleren Abschnitts überführt werden, wobei es diese Form dem gesamten Formgebungsteil aufprägt. Zugleich besitzt es im verformten Zustand ausreichende Elastizität, um den weiter oben genauer erläuterten allgemeinen Anforderungen an das erfindungsgemäße Formgebungsteil zu genügen.

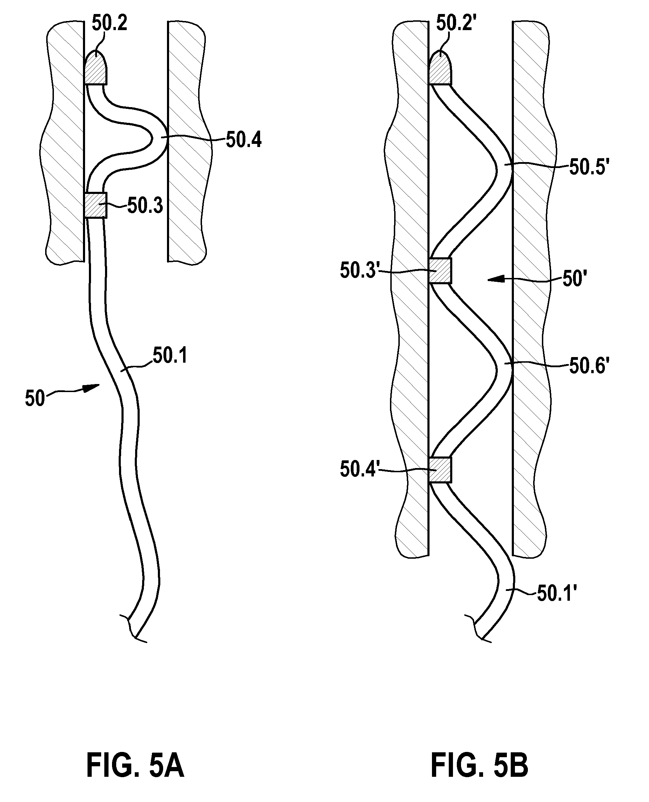

Fig. 5A und 5B zeigen zwei Konfigurationen von Elektrodenleitungsanordnungen, die mit Formgebungsteilen der vorstehend beschriebenen Art realisiert werden können. Fig. 5A zeigt eine Elektrodenleitungsanordnung 50 mit einer Elektrodenleitung (Elektrode) 50.1, die eine Spitzenelektrode 50.2 und eine Ringelektrode 50.3 als Elektrodenpole hat und die durch eine zwischen den beiden Elektrodenpolen 50.2, 50.3 auf die Leitung aufgeschrumpftes Formgebungsteil 50.4 im Endabschnitt V-förmig verformt ist. Hierdurch verspannt sich die Elektrodenleitungsanordnung 50 zwischen den gegenüberliegenden Wandungen eines Gefäßes, was wiederum in erwünschter Weise dazu führt, dass die Elektrodenpole 50.2 und 50.3 zuverlässigen Wandkontakt haben und somit ihre Stimulations- und/oder Sensing-Aufgabe dauerhaft zuverlässig erfüllen können.

Fig. 5B zeigt als Abwandlung dieser Konfiguration eine weitere Elektrodenleitungsanordnung 50', die eine dreipolige Elektrodenleitung 50.1' mit den Elektrodenpolen 50,2', 50.3' und 50.4' und zwei Formgebungsteile 50.5', 50.6' umfasst. Beide Formgebungsteile sind jeweils zwischen zweien der drei Elektrodenpole um die entsprechenden Abschnitte der Elektrode 50.1 gelegt und dort mit der Elektrode dicht verklebt. Es ist zu erkennen, dass die Elektrodenanordnung bei dieser Ausführungsform eine im Endabschnitt insgesamt wellenförmige Gestalt angenommen hat, wobei diese Gestalt wiederum das erwünschte Ergebnis hat, dass alle Elektrodenpole 50.2', 50.3' und 50.4' zuverlässigen Wandkontakt haben.

Das Aufschrumpfen der Formgebungsteile auf die Elektrode wird in an sich bekannter Weise ausgeführt. Formgebungsteile aus Silikon werden vor Montage aufgequollen (z.B. mit Heptan), sodass die Teile über die Wendel in die gewünschte Position geschoben werden können - Heptan entweicht nach und nach, das Silikon zieht sich wieder zusammen und schrumpf so auf die Wendel. Falls Kunststoffverstärkungsteile in diesem Silikonteil integriert sind kann dieses Verfahren nur begrenzt eingesetzt werden, da Kunststoff nicht quillt und es zu einem Ablösen der beiden Materialien voneinander kommen kann. Eine weitere Möglichkeit ist das kurzzeitige Aufweiten des Innendurchmessers des Formgebungsteils durch Druckluft. Die Enden werden abgedichtet, Druckluft wird eingefüllt, das Teil kann auf oder über größere Durchmesser montiert und abgelegt werden.

Bei einer weiteren Variante wird das Teil mechanisch aufgeweitet z.B. durch mehrere innen liegende Drähte oder Halbschalen. Somit kann das Teil auch hier auf oder über größere Durchmesser montiert und abgelegt werden.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Implantierbares gebogenes Formgebungsteil (10 - 40) zur äußeren Formung einer implantierbaren Elektrodenleitung (50.1; 50.1') oder eines Katheters, wobei das Formgebungsteil ein durchgehendes erstes Lumen (10a; 20a; 30a; 40a) zum Durchgang eines Abschnitts der Elektrodenleitung oder des Katheters hat,
wobei das Formgebungsteil als Spritzgussteil ausgebildet ist oder mindestens einen Spritzguss-Abschnitt aufweist und innerhalb der Wandung oder an der Innenwand ein langgestrecktes, steifes aber biegsames Biegungs-Einprägeelement (11; 21; 31; 41) fixiert ist.

2. Formgebungsteil nach Anspruch 1, ausgebildet als im Gebrauchszustand U-förmiges oder V-förmiges Teil mit verrundeter Spitze.

3. Formgebungsteil nach Anspruch 1 oder 2, wobei das Spritzgussteil oder der Spritzguss-Abschnitt aus Silikon mit geringer Shore-Härte, insbesondere von 50 Shore oder weniger, ausgebildet ist.

4. Formgebungsteil nach einem der vorangehenden Ansprüche, wobei an beiden Enden das erste Lumen (10a) in seinem Durchmesser derart aufgeweitet ist (10b; 20c; 30d), dass Platz für eine innenliegende Klebstoffschicht geschaffen ist.

5. Formgebungsteil nach Anspruch 4, ausgebildet als extrudierter Silikonschlauch (20'; 30') mit umspritzten Enden, die den aufgeweiteten Querschnitt des ersten Lumens bestimmen, welcher mindestens ein zweites Lumen (20b; 30b) hat, in dem das Biegungs-Einprägeelement (21; 31) angeordnet ist.

6. Formgebungsteil nach Anspruch 5, wobei das zweite Lumen (20b; 30b) durchgehend ist.

7. Formgebungsteil nach einem der vorangehenden Ansprüche, wobei das Biegungs-Einprägeelement (11; 21) ein federnd steifer gebogener Kunststoffstab oder -streifen ist.

8. Formgebungsteil nach Anspruch 7, wobei das Biegungs-Einprägeelement ein Stab aus einem Memory-Material ist.

9. Formgebungsteil nach einem der Ansprüche 1 bis 6, wobei das Biegungs-Einprägeelement (31) ein vorgespannter, durch Tempern mit einer Krümmung versehener Kunststoffstab oder -streifen ist.

10. Formgebungsteil nach einem der vorangehenden Ansprüche, mit mehreren Biegungs-Einprägeelementen, die insbesondere jeweils in einem vorgeformten zweiten Lumen angeordnet sind.

11. Elektrodenleitungsanordnung (50; 50') mit einer implantierbaren Elektrodenleitung (50.1; 50.1') und mindestens einem Formgebungsteil nach einem der vorangehenden Ansprüche, welches einen distalen Abschnitt der Elektrodenleitung umgibt.

12. Elektrodenleitungsanordnung nach Anspruch 11, wobei das Formgebungsteil (50.4; 50.5', 50.6') einen Abschnitt zwischen zwei Elektrodenpolen (50.2, 50.3; 50.2', 50.3', 50.4') vollständig umgibt, derart, dass ein erstes Ende des Formgebungsteils direkt an einen ersten Elektrodenpol anstößt und ein zweites Ende des Formgebungsteils direkt an einen zweiten Elektrodenpol anstößt.

13. Elektrodenleitungsanordnung nach Anspruch 11 oder 12, wobei zwei oder mehr U-förmige oder V-förmige Formgebungsteile (50.5', 50.6') in Längsrichtung hintereinander auf der Elektrodenleitung (50.1; 50.1') optional mit entgegengerichteter Krümmung derart angeordnet sind, dass ein distaler Endabschnitt der Elektrodenleitung insgesamt einen im Wesentlichen S-förmigen, Z-förmigen, J-förmigen, wellenförmigen oder zickzackförmigen Verlauf aufgeprägt hat.

14. Katheteranordnung mit einem Katheter und mindestens einem Formgebungsteil (10) nach einem der Ansprüche 1 bis 10.
